(19) Europäisches Patentamt
European Patent Office
Office européen des brevets

(11) EP 0 709 387 B1

(12) EUROPÄISCHE PATENTSCHRIFT

(45) Veröffentlichungstag und Bekanntmachung des Hinweises auf die Patenterteilung:
**03.03.1999 Patentblatt 1999/09**

(51) Int. Cl.$^6$: **C07D 493/04**, A61K 6/083
// (C07D493/04, 321:00, 317:00)

(21) Anmeldenummer: **95250258.1**

(22) Anmeldetag: **25.10.1995**

# (54) Bicycloaliphatische 2-Methylen-1,3-dioxepane

Bicycloaliphatic 2-methylene-1,3-dioxepanes

2-Méthylène-1,3-dioxépanes bicycloaliphatiques

(84) Benannte Vertragsstaaten:
**AT CH DE FR GB IT LI SE**

(30) Priorität: **26.10.1994 DE 4439485**

(43) Veröffentlichungstag der Anmeldung:
**01.05.1996 Patentblatt 1996/18**

(73) Patentinhaber: **IVOCLAR AG**
**FL-9494 Schaan (LI)**

(72) Erfinder:
- **Moszner, Norbert Prof. Dr.**
  **LI-9492 Eschen (LI)**
- **Völkel, Thomas Dr.**
  **D-88131 Lindau (DE)**
- **Rheinberger, Volker Dr.**
  **LI-9490 Vaduz (LI)**
- **Salz, Ulrich Dr.**
  **D-88131 Lindau (DE)**

(74) Vertreter: **UEXKÜLL & STOLBERG**
**Patentanwälte**
**Beselerstrasse 4**
**22607 Hamburg (DE)**

(56) Entgegenhaltungen:
**EP-A- 0 206 074** **EP-A- 0 209 700**
**GB-A- 2 107 341**

- **MACROMOLECULAR CHEMISTRY AND PHYSICS, Bd. 196, Nr. 2, Februar 1995 Seiten 567-572, T. SCHULZE ET AL. 'Crosslinking copolymerization of cyclic ketenacetals with low shrinkage in volume'**
- **PATENT ABSTRACTS OF JAPAN vol. 10 no. 235 (C-366) ,14.August 1986 & JP-A-61 069704 (SANKIN KOGYO KK) 10.April 1986,**
- **PATENT ABSTRACTS OF JAPAN vol. 10 no. 235 (C-366) ,14.August 1986 & JP-A-61 069706 (SANKIN KOGYO KK) 10.April 1986,**
- **MACROMOLECULES, Bd. 15, Nr. 3, 1982 Seiten 711-714, W. J. BAILEY ET AL. 'Free Radical Ring-Opening Polymerization ...'**
- **JOURNAL OF POLYMER SCIENCE, Bd. 20, Nr. 9, 1982 Seiten 3021-3030, W. J. BAILEY ET AL. 'Synthesis of ...'**
- **JOURNAL OF POLYMER SCIENCE - POLYMER LETTERS EDITION, Bd. 21, 1983 Seiten 373-380, T. ENDO ET AL. 'Photoinitiated Ring-Opening Polymerization ...'**
- **JOURNAL OF MACROMOLECULAR SCIENCE - CHEMISTRY, Bd. A21, Nr. 8-9, 1984 Seiten 979-995, W. J. BAILEY ET AL. 'Synthesis of ...'**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.7/3.6

## Beschreibung

Die vorliegende Erfindung betrifft neuartige bicycloaliphatische 2-Methylen-1,3-dioxepane, die sich unter Ringöffnung mit nur sehr geringem Volumenschrumpf radikalisch und kationisch polymerisieren lassen. Die Erfindung betrifft ferner die Verwendung dieser Dioxepane zur Herstellung von Komposit- bzw. Dentalmaterialien.

Es ist im Stand der Technik bekannt, daß bei der Polymerisation von Monomeren oder Monomergemischen in der Regel ein unterschiedlich stark ausgeprägter Volumenschrumpf beobachtet wird R.M. Luck, R.k. Sadhir, Expanding Monomers - Synthesis, Characterization and Applications; CRS Press (1992) 4). Der Volumenschrumpf ($V_{schr}$) ist durch die Formel

$$V_{schr} = (V_m - V_p) / V_m,$$

definiert, wobei $V_m$ für das Monomervolumen und $V_p$ für das Polymervolumen stehen.

Bei Formkörpern wirkt sich dieser Volumenschrumpf äußerst nachteilig auf die Dimensionsstabilität sowie die mechanischen Eigenschaften der Formkörper aus, während bei Adhäsivsystemen bzw. Klebeverbindungen die Haftungseigenschaften bzw. die Verbundfestigkeit negativ beeinflußt wird.

Es ist eine Reihe von Monomersystemen bekannt, wie zum Beispiel Epoxid-Harze (B. Ellis; Chemistry and Technology of Epoxy Resins, Blackie Acad. & Profess., London 1992) oder doppelringöffnende Monomere auf der Basis von Spiroorthoestern bzw. Spiroorthocarbonaten (R.F. Brady; J. Macromol. Sci.-Rev. Macromol. Chem. Phys. C32 (1992) 135), die unter geringer bzw. ohne Volumenkontraktion polymerisieren. Der Nachteil dieser Monomersysteme besteht allerdings darin, daß die Photohärtung bei Raumtemperatur, wie zum Beispiel bei den Epoxidharzen, nur sehr langsam abläuft, was für die schnelle Verarbeitbarkeit dieser Werkstoffe von Nachteil ist. Ferner lassen sich diese Monomersysteme bzw. die daraus gebildeten Polymere aus toxikologischen Gründen im medizinischen bzw. zahnmedizinischen Bereich nicht verwenden. Die Synthese von Spiroorthoestern bzw. von Spiroorthocarbonaten ist im übrigen äußerst aufwendig und die Ringöffnungstendenz dieser Verbindungen bei der radikalischen Photoinitiierung ist nur sehr gering, so daß sich diese Materialien nur sehr eingeschränkt verwenden lassen und sich ausschließlich für spezielle Anwendungen eignen, bei denen die Nachteile im Hinblick auf die Verarbeitbarkeit und die toxikologischen Eigenschaften nicht zum Tragen kommen und die aufwendigere Herstellung der Polymere aufgrund der für den Spezialfall gewünschten Eigenschaften in Kauf genommen wird.

2-Methylen-1,3-dioxepan-Derivate sind im Stand der Technik als relativ einfach zugängliche Monomersysteme bekannt, die einer radikalischen Ringöffnungspolymerisation unterzogen werden können. So ist das unsubstituierte 2-Methylen-1,3-dioxepan zum Beispiel ausgehend von Chloracetaldehyddimethylacetal und 1,4-Butandiol über zwei Syntheseschritte in hohen Ausbeuten zugänglich (E. Taskinen, M.-L. Pentikainen, Tetrahedron **34** (1978) 2365) (vgl. Fig. 1).

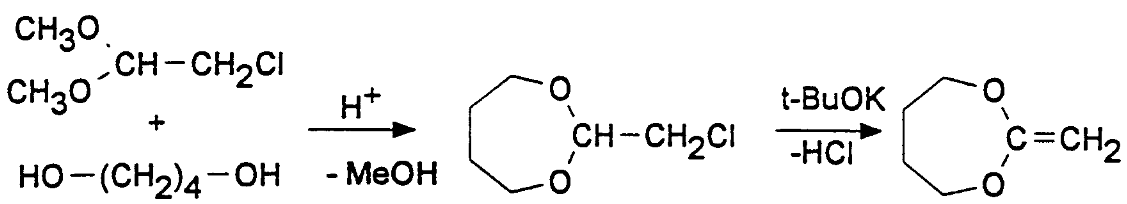


Fig. 1: Darstellung von 2-Methylen-1,3-dioxepan

Bailey et al. beschrieben erstmals die thermisch initiierte radikalische Ringöffnungspolymerisation von 2-Methylen-1,3-dioxepan (W.J. Bailey et al., J. Polym. Sci., Polym. Chem. Ed. **20** (1982) 3021; W.J. Bailey et al., Proc. IUPAC Macromol. Symp. 28th. (1982) 214; Chem. Abstr. **99** (1983) 158955u), wobei mit Hilfe von $^{13}$C-NMR- und IR-Spektroskopie gezeigt werden konnte, daß die Polymerisation vollständig unter Ringöffnung abläuft (Fig. 2). Das ausgehend von 2-Methylen-1,3-dioxepan gebildete Homopolymer weist einen gummi- bzw. wachsartigen Charakter auf.

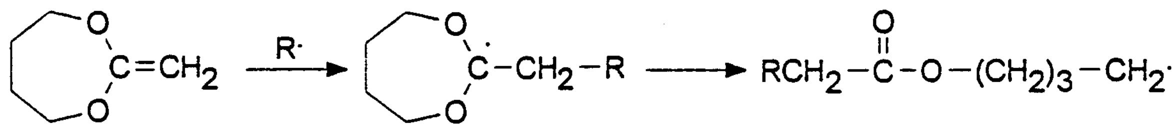

Fig. 2: Ringöffnung von 2-Methylen-1,3-dioxepan

Bei Raumtemperatur (25 °C) kann 2-Methylen-1,3-dioxepan auch in Gegenwart von 2-Ethylanthrachinon bzw. Benzoinisopropylester als Photoinitiator unter vollständiger Ringöffnung durch Lichtinitiierung polymerisiert werden (T. Endo, M. Okawara, W.J. Bailey; J. Polym. Sci., Polym. Lett. Ed. **21** (1983) 373).

Für die weitere Untersuchung der Ringöffnungstendenz von 2-Methylen-1,3-dioxepanen wurden von Bailey et al. auch 5,6-Benzo-2-methylen-1,3-dioxepan und eine cis-trans-Isomermischung von 4,7-Dimethyl-2-methylen-1,3-dioxepan hergestellt (Bailey et al., Macromolecules **15** (1982) 711) (vgl. Fig. 3). Zur Bildung eines festen Polymers aus Verbindung 5,6-Benzo-2-methylen-1,3-dioxepan waren drastische Reaktionsbedingungen (120 °C, 15 Stunden in Gegenwart von 2,5 Mol.-% Di-t-butylperoxid (DTBP)) notwendig, unter vergleichbaren Bedingungen lieferte der Einsatz von Verbindung 4,7-Dimethyl-2-methylen-1,3-dioxepan (120 °C, 72 Stunden, 2 Mol.-% DTBP) selbst nach verlängerter Reaktionsdauer lediglich ein viskoses Polymer. In beiden Fällen konnte eine 100 %-ige Ringöffnung spektroskopisch nachgewiesen werden.

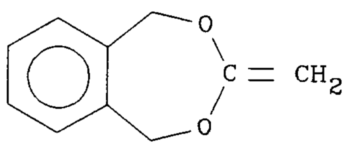

5,6-Benzo-2-methylen-1,3-dioxepan

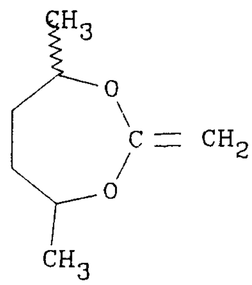

4,7-Dimethyl-2-methylen-1,3-dioxepan

Fig. 3:

Im Stand der Technik ist ferner die Copolymerisation von 2-Methylen-1,3-dioxepan-Derivaten bekannt. So wurden zum Beispiel die Verbindung 2-Methylen-1,3-dioxepan mit Vinylgruppen-enthaltenden Monomeren, wie zum Beispiel Styrol und Methylmethacrylat zur Reaktion gebracht (W.J. Bailey et al., J. Macromol. Sci.-Chem. **A21** (1984) 979).

In der JP-A-61069704 wird die Verwendung des sehr aufwendig herzustellenden, kristallinen 5,6-Benzo-2-methylen-1,3-dioxepan als schrumpfungsarme und die Dentinhaftung fördernde Monomerkomponente eines Dentalmaterials beschrieben. Der Polymerisationsschrumpf ist für daraus hergestellte Dentalmaterialien nicht angegeben, und die Dentinhaftungswerte sind im Vergleich zu anderen, im Stand der Technik bekannten Systemen (D.H. Retief, R.S. Russell; Am. J. Dent. **7** (1994) 43), mit maximal 5,3 MPa nur äußerst mangelhaft. Dentalmaterialien auf Basis von 2-Methylen-1,3-dioxepan werden in JP-A-61069706 offenbart.

Im Stand der Technik sind bislang keine Verbindungen oder Systeme bekannt, bei deren Polymerisation nur ein äußerst geringer Volumenschrumpf eintritt und die sich aufgrund ihrer toxikologischen Unbedenklichkeit sowie ihrer allgemeinen physikalischen Eigenschaften in besonders bevorzugter Weise zur Herstellung von Komposit- bzw. Dentalmaterialien eignen.

Es ist daher die Aufgabe der vorliegenden Erfindung, neue 2-Methylen-1,3-dioxepane als Ausgangsstoffe zur Herstellung von Polymeren bzw. Copolymeren, vorzugsweise von Dentalmaterialien, zur Verfügung zu stellen, die auf einfache Weise hergestellt werden können, sich bei Raumtemperatur in kurzer Zeit härten lassen, nur unter äußerst geringem Volumenschrumpf polymerisieren und die als Dentalmaterialien gute physikalische Eigenschaften aufweisen.

Erfindungsgemäß wird diese Aufgabe durch die bicycloaliphatischen 2-Methylen-1,3-dioxepane nach den Ansprüchen 1 bis 8 gelöst.

Gemäß der vorliegenden Erfindungen werden bicycloaliphatische 2-Methylen-1,3-dioxepane der allgemeinen Formel

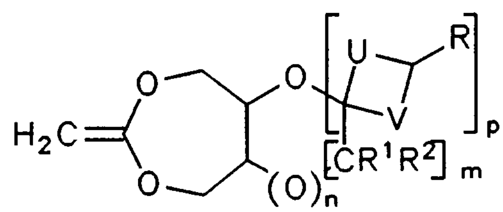

(I)

zur Verfügung gestellt, wobei

n gleich 1 ist, und p gleich 0 und m gleich 0 ist, oder
p gleich 1 und m gleich 0 ist,
U und V jeweils Sauerstoff oder $(CH_2)_q$ bedeuten, wobei q eine ganze Zahl im Bereich von 0 bis 6 ist,
R, $R^1$ und $R^2$ jeweils für Wasserstoff, eine Alkylgruppe, eine Alkenylgruppe, eine Arylgruppe oder einen cycloaliphatischen Rest stehen und jeweils einen weiteren Substituenten aus der Gruppe bestehend aus Alkyl, Vinyl, Acryl, Acryloxy, Methacryl, Methacryloxy, $COOR^3$, $CONR^3_2$ und $OR^3$ enthalten können und wobei $R^3$ für eine Alkylgruppe und/oder Arylgruppe steht, wobei einer der Reste R, $R^1$ oder $R^2$ mit einem zweiten 2-Methylen-1,3-dioxepanring der Formel (I) verknüpft sein kann oder $R^1$ und $R^2$ für eine Methylengruppe steht.

Das mit der Methylengruppe direkt verknüpfte bicycloaliphatische Ringsystem der erfindungsgemäßen 2-Methylen-1,3-dioxepane stellt die strukturelle Einheit dar, die für die ringöffnende Polymerisation verantwortlich ist. Die weiteren Struktureinheiten, die mit dem Ring verknüpft sind, der die Methylengruppe nicht trägt, bzw. darin enthalten sind, d.h. der Spirosubstituent bzw. die Gruppe $(CR^1R^2)_m$, bestimmen im wesentlichen die physikalischen Eigenschaften der Dioxepane bzw. der daraus hergestellten Polymere bzw. Copolymere.

Unter Alkyl und Alkylen werden im Zusammenhang mit den bicycloaliphatischen 2-Methylen-1,3-dioxepanen bevorzugt solche gesättigte und ungesättigte aliphatische Kohlenwasserstoff-Gruppen verstanden, die 1 bis 10, vorzugsweise 1 bis 6 und besonders bevorzugt 1 bis 4 Kohlenstoffatome enthalten, wobei diese Gruppen sowohl linear als auch verzweigt sein können. Mit Aryl sind Reste gemeint, die insbesondere 6 bis 14 Kohlenstoffatome aufweisen und wie oben angegeben substituiert sein können. Die Reste R, $R^1$, $R^2$ und $R^3$ können jeweils gleich oder verschieden sein. Gemäß einer Ausführungsform der Erfindung können R, $R^1$, $R^2$ und/oder $R^3$ für cycloaliphatische Reste stehen, die bis zu 12 Kohlenstoffatome enthalten und gegebenfalls über Brückensubstituenten, wie z.B. $-(CH_2)_n-CO_2-$, mit dem 2-Methylen-1,3-dioxepangerüst verbunden sein können.

Gemäß einer bevorzugten Ausführungsform der Erfindung wird ein bicycloaliphatisches 2-Methylen-1,3-dioxepan der Formel (I) zur Verfügung gestellt, bei dem n gleich 1 ist und p gleich 0 ist und wobei m, q, R, $R^1$, $R^2$ und $R^3$ die oben angegebene Bedeutung haben. Diese Klasse von Dioxepanen kann durch die allgemeine Formel (II)

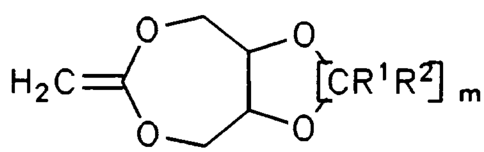

(II)

dargestellt werden.

Bevorzugte Vertreter der bicycloaliphatischen Dioxepane gemäß der allgemeinen Formel (II) enthalten ausschließlich Sauerstoff als Substituenten des bicycloaliphatischen Dioxepan-Gerüsts, und die Reste $R^1$ und $R^2$ werden vorzugsweise aus der Gruppe bestehend aus Wasserstoff, Methyl, Ethyl, Cyclohexyl, Norborn-1-en-5-yl und Phenyl ausgewählt, wobei $R^1$ und $R^2$ jeweils gleich oder verschieden sein können und einen weiteren Substituenten R tragen können, der die oben angegebene Bedeutung hat. In besonders bevorzugter Weise stehen die Substituenten $R^1$ und $R^2$ für eine Methylengruppe.

Exemplarische Vertreter der bicycloaliphatischen Dioxepane der allgemeinen Formel (II) sind in Tabelle 1 dargestellt.

<u>Tabelle 1:</u> Bicycloaliphatische 2-Methylen-1,3-dioxepane der allgemeinen Formel (II)

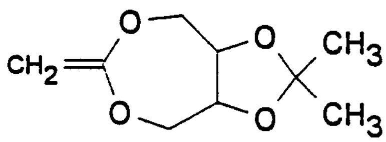

<u>1</u>

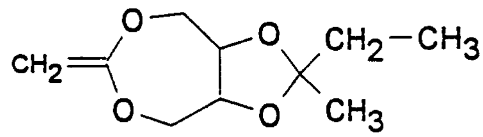

<u>2</u>

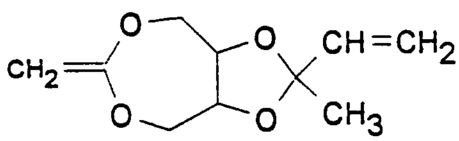

<u>3</u>

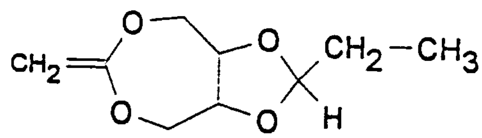

<u>4</u>

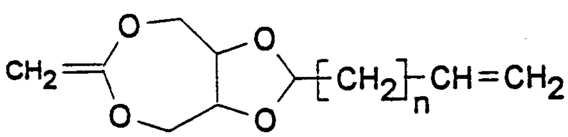

<u>5</u>

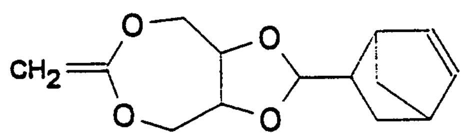

<u>6</u>

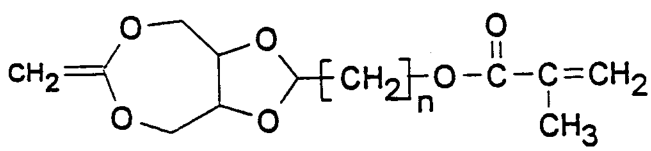

<u>7</u>

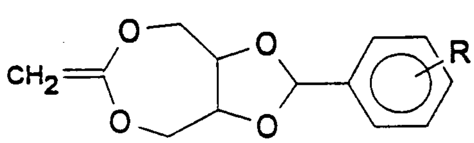

<u>8</u>

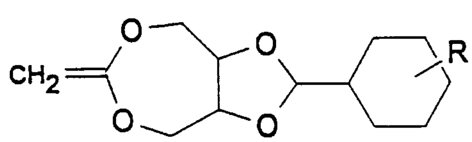

<u>9</u>

Tabelle 1 - Fortsetzung

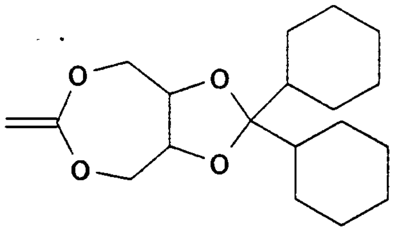

10

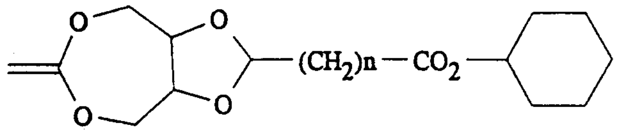

11

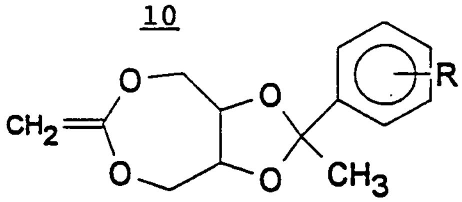

12

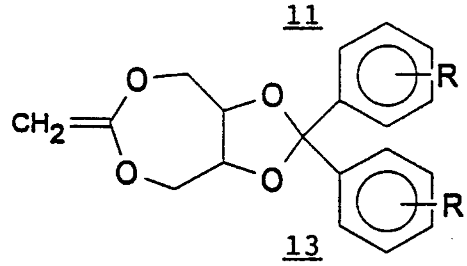

13

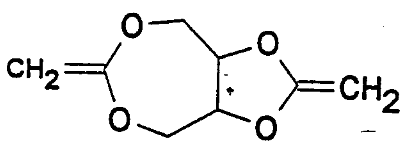

14

Ferner sind ausschließlich Sauerstoff-enthaltende bicycloaliphatische 2-Methylen-1,3-dioxepane der allgemeinen Formel (II) bevorzugt, bei denen $R^1$ Wasserstoff, Alkyl oder Aryl bedeutet und $R^2$ für einen Substituenten der Formel $(CH_2)_rR$ steht, wobei r eine ganze Zahl im Bereich von 0 bis 4 ist und R aus der Gruppe bestehend aus Vinyl, Acryloxy und Methacryloxy ausgewählt ist. Derartige Verbindungen, die über die ringöffnenden Dioxepane weitere polymerisationsfähige olefinische Gruppen enthalten, sind von besonderem Vorteil zur Herstellung von polymeren Werkstoffen mit verbesserten mechanischen Eigenschaften, die während der Polymerisation jedoch keinem Volumenschrumpf unterliegen. In besonders bevorzugter Weise können die bicycloaliphatischen Dioxepane der allgemeinen Formel (II), bei denen m gleich 1 ist, über einen Phenylrest ($R^2$) mit einer weiteren 2-Methylen-1,3-dioxepan-Gruppe der allgemeinen Formel (II) verknüpft sein. Der Rest $R^1$ wird in diesem Falle aus der Gruppe bestehend aus Wasserstoff, Alkyl oder Aryl ausgewählt. Exemplarische Vertreter dieser Gruppe von Dioxepanen sind in Tabelle 2 dargestellt. Diese lassen sich sowohl radikalisch als auch kationisch bei vermindertem Polymerisationsschrumpf vernetzen. Gemäß der vorliegenden Erfindung kann ferner ein epoxygruppenhaltiges Dioxepan zur Verfügung gestellt werden, das ebenfalls keinem Volumenschrumpf unterliegt. Die Epoxygruppe eignet sich in besonderem Maße zur weiteren Funktionalisierung bzw. Vernetzung (vgl. Fig. 4)

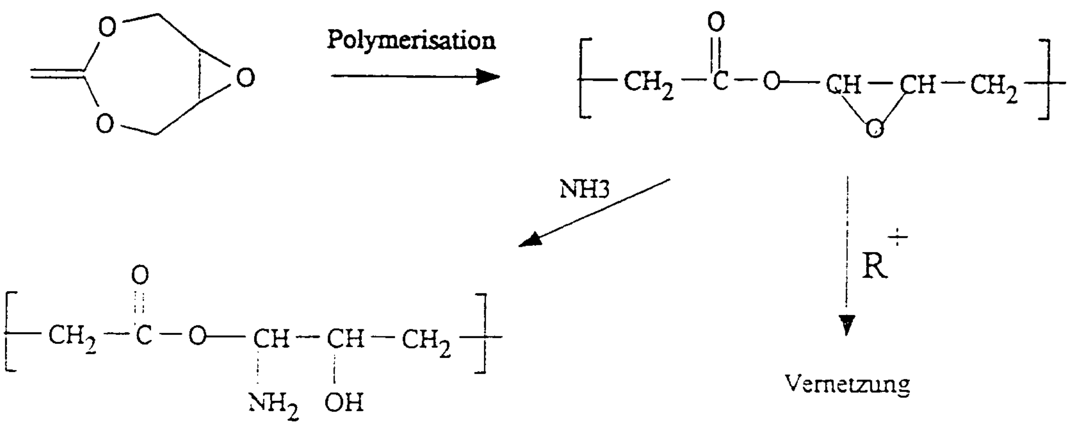


Fig. 4: Vernetzung bzw. Funktionalisierung über eine Epoxygruppe

Im Rahmen der vorliegenden Erfindung werden ferner bicycloaliphatische Dioxepane zur Verfügung gestellt, die an ihrem Grundgerüst einen weiteren, spiroverknüpften Ring tragen. Diese Dioxepane entsprechend der allgemeinen Formel (III)

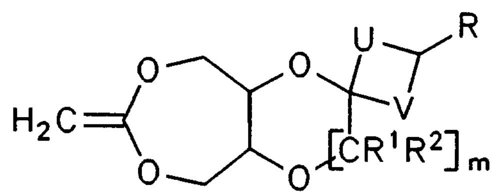

(III),

wobei m, q, U, V, R, $R^1$, $R^2$ und $R^3$ die oben angegebene Bedeutung haben.

Gemäß einer besonders bevorzugten Ausführungsform werden bicycloaliphatische 2-Methylen-1,3-dioxepan-Derivate zur Verfügung gestellt, die einen spiroverknüpften Cyclohexylring enthalten, wobei auch bei dieser Gruppe von Dioxepan-Derivaten die Verknüpfung mit einer zweiten 2-Methylen-1,3-dioxepan-Einheit zur Vernetzung von Vorteil ist. Beispielhafte Vertreter dieser Dioxepane sind in den Tabellen 2 und 3 dargestellt.

Die bicycloaliphatischen 2-Methylen-1,3-dioxepan-Derivate der vorliegenden Erfindung sind in einfacher Weise zugänglich, wie z.B. über die in Fig. 5 dargestellte, vier Schritte umfassende Synthese. So kann beispielsweise zunächst im ersten Schritt eine Umacetalisierung von 2-Bromacetaldehyddiethylacetal mit cis-2-Buten-1,4-diol erfolgen. Im Anschluß daran wird die Doppelbindung des gebildeten Dioxepans mit Kaliumpermanganat oder einem äquivalenten Oxidationsmittel zum cis-Diol oxidiert, und durch Umsetzung mit einem entsprechenden Keton oder Aldehyd wird im dritten Schritt das entsprechende Acetal gebildet. Nach HBr-Abspaltung erhält man in der vierten Stufe das entsprechende bicycloaliphatische 2-Methylen-1,3-dioxepan-Derivat (Fig. 5).

<u>Tabelle 2:</u> Bicycloaliphatische 2-Methylen-1,3-dioxepan-Derivate der allgemeinen Formel (II) und (III) mit mindestens zwei 2-Methylen-1,3-dioxepaneinheiten

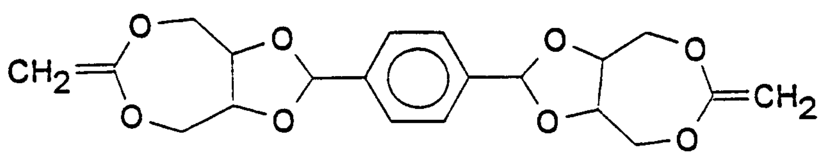

15

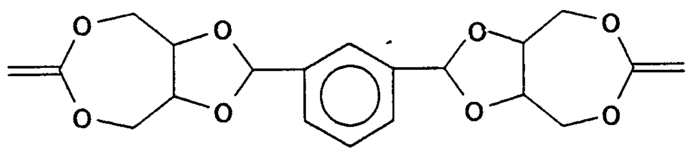

16

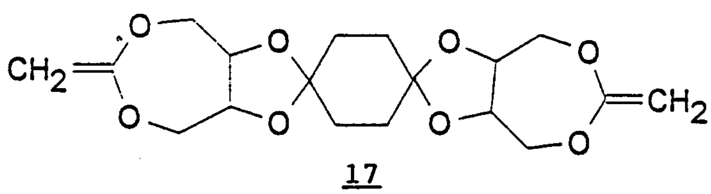

17

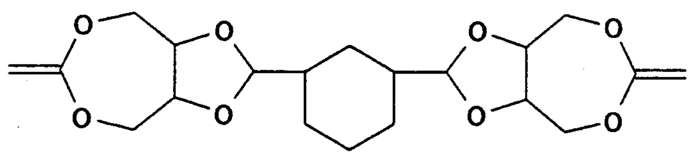

18

Tabelle 3: Bicycloaliphatische 2-Methylen-1,3-dioxepan-Derivate der allgemeinen Formel (III)

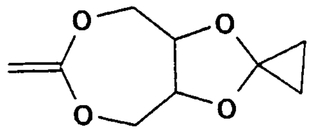

19

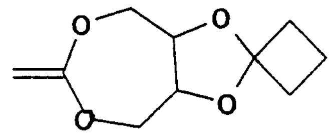

20

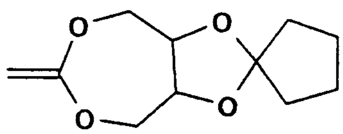

21

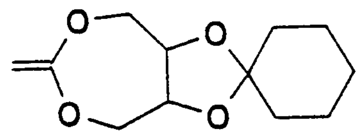

22

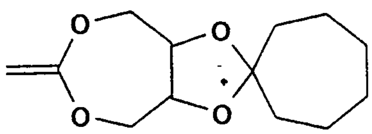

23

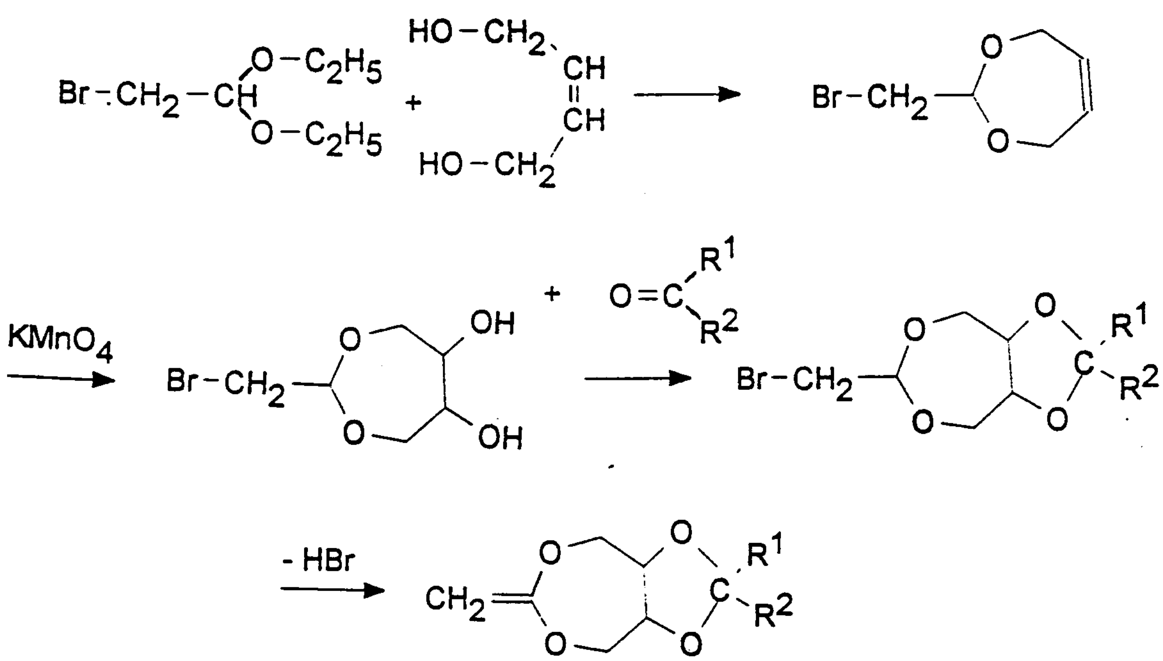

Fig. 5: Beispielhafte Darstellung der 2-Methylen-1,3-dioxepane der allgemeinen Formel (II)

In analoger Weise können ausgehend von den entsprechenden Dialkylketalen oder Dialkylacetalen die bicycloaliphatischen Dioxepan-Derivate der allgemeinen Formel (III) durch Umacetalisierung erhalten werden (Fig. 6).

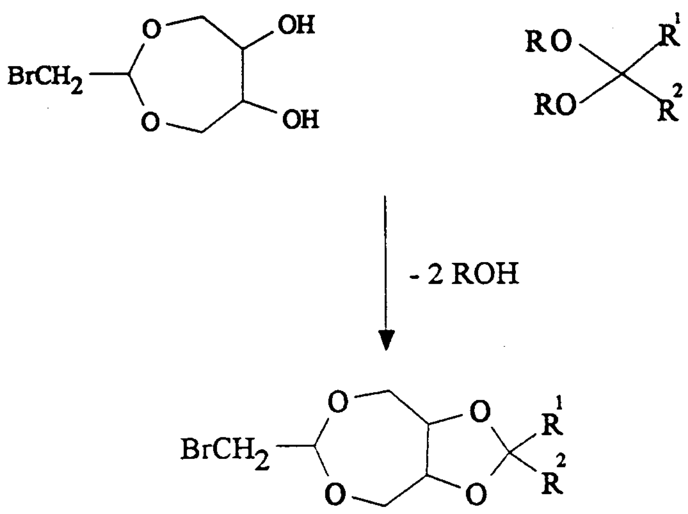


Fig. 6: Beispielhafte Darstellung der 2-Methylen-1,3-dioxepane der allgemeinen Formel (III)

Es hat sich überraschenderweise gezeigt, daß die bicycloaliphatischen 2-Methylen-1,3-dioxepane der vorliegenden Erfindung bei der Polymerisation einen deutlich niedrigeren Volumenschrumpf aufweisen als nichtsubstituierte 2-Methylen-1,3-dioxepane im Stand der Technik. Die erfindungsgemäßen Verbindungen dienen als Ausgangsstoffe zur Herstellung von Polymeren und/oder Copolymeren, wie z.B. von Dentalmaterialien. Sie können auf einfache Weise hergestellt werden, lassen sich bei Raumtemperatur radikalisch oder kationisch in Gegenwart geeigneter Photoinitiatoren, thermisch oder in Gegenwart von Radikalinitiatoren oder kationischen Initiatoren polymerisieren. Dabei tritt nur ein äußerst geringer Volumenschrumpf auf.

Beispielsweise läuft die thermisch initiierte Substanzhomopolymerisation von 4-Methylen-9,9-dimethyl-3,5,8,10-tetraoxabicyclo[5.3.0]decan mit 2,5 Mol-% DTP (120 °C, 16 Stunden) unter einer Volumenkontraktion von nur 2,1 % ab. Im Vergleich zu der von Bailey et al. beschriebenen Polymerisation des unsubstituierten 2-Methylen-1,3-dioxepans ist der Volumenschrumpf des entsprechenden Dioxepan-Derivats der vorliegenden Erfindung somit um einen Faktor von ca. 3 geringer als für Dioxepane im Stand der Technik. Mit Hilfe der $^{13}C$-NMR-Spektroskopie konnte nachgewiesen werden, daß die Homopolymerisation des erfindungsgemäßen 4-Methylen-9,9-dimethyl-3,5,8,10-tetraoxa-bicyclo[5.3.0]decan unter vollständiger Ringöffnung abläuft. Einen ebenso geringen Volumenschrumpf weisen auch die aus den bicyclischen 2-Methylen-1,3-dioxepanen 4-Methylen-9-ethyl-9-methyl-3,5,8,10-tetraoxa-bicyclo[5.3.0]decan, 4-Methylen-9-methyl-9-vinyl-3,5,8,10-tetraoxa-bicyclo[5.3.0]decan und 4-Methylen-9-ethyl-3,5,8,10-tetraoxa-bicyclo[5.3.0]decan hergestellten Polymere auf (vgl. Tabelle 4).

Tabelle 4

| Schrumpfwerte für aus erfindungsgemäßen bicyclischen 2-Methylen-1,3-dioxepanen nach Photopolymerisation (vgl. Beispiel 8) erhaltene Polymere | |
|---|---|
| Monomere (Verweis auf Beispiel) | Schrumpfwert (%) |
| Methylen-9,9-dimethyl-3,5,8,10-tetraoxa-bicyclo[5.3.0]decan (1) (vgl. Beispiel 1) | 6,2 |
| 4-Methylen-9-ethyl-9-methyl-3,5,8,10-tetraoxa-bicyclo[5.3.0]decan (2) (vgl. Beispiel 2) | 7,5 |
| 4-Methylen-9-methyl-9-vinyl-3,5,8,10-tetraoxa-bicyclo[5.3.0]decan (3) (vgl. Beispiel 3) | 6,2 |
| 4-Methylen-9-ethyl-3,5,8,10-tetraoxa-bicyclo[5.3.0]decan (4) (vgl. Beispiel 4) | 4,8 |
| Vergleich: Triethylenglykoldimethacrylat | 14,2 |

Tabelle 4 (fortgesetzt)

| Schrumpfwerte für aus erfindungsgemäßen bicyclischen 2-Methylen-1,3-dioxepanen nach Photopolymerisation (vgl. Beispiel 8) erhaltene Polymere | |
|---|---|
| Monomere (Verweis auf Beispiel) | Schrumpfwert (%) |
| Vergleich: 1,10-Decandioldimethacrylat | 10,1 |

Gemäß der vorliegenden Erfindung läßt sich ferner zeigen, daß auch bei der Copolymerisation der 2-Methylen-1,3-dioxepan-Derivaten, wie zum Beispiel mit Dimethacrylaten (z.B. propoxyliertes Bis-GMA) im Vergleich zu reinen Dimethacrylatmischungen ein signifikant reduzierter Polymerisationsschrumpf erfolgt. Die bicycloaliphatischen 2-Methylen-1,3-dioxepane der allgemeinen Formel (I) sind besonders zur Herstellung dimensionsstabiler Formkörper und insbesondere zur Anwendung in bzw. als Dentalmaterialien geeignet. Neben dem relativ geringen Volumenschrumpf zeichnen sich die aus den erfindungsgemäßen 2-Methylen-1,3-dioxepanen hergestellten Polymere bzw. Copolymere durch die einfache Herstellung, einfache Reinigung und leichte Modifizierbarkeit aus. Die Eigenschaften der Polymere lassen sich damit durch die Wahl der Substituenten R, $R^1$, $R^2$ und/oder $R^3$ im Monomer sowie über zusätzliche Vernetzung auf einfache Weise gezielt steuern. Insbesondere hat sich gezeigt, daß sich die Dioxepan-Derivate der vorliegenden Erfindung auch bei Raumtemperatur in relativ kurzer Zeit polymerisieren lassen, wobei die Herstellung der Polymere durch Bestrahlung im UV oder im sichtbaren Bereich erfolgen kann.

Die bicycloaliphatischen Dioxepane der vorliegenden Erfindung eignen sich besonders zur Herstellung von schrumpfungsarmen Kompositmaterialien bzw. von Dentalwerkstoffen. Insbesondere sind die 2-Methylen-1,3-dioxepane der vorliegenden Erfindung als Füllungsmaterial und Dentaladhäsiv bevorzugt geeignet. Vorteilhafterweise werden zusätzlich Füllstoffe, Monomere als Vernetzer und/oder Katalysatoren verwendet, wobei als Füllstoffe pyrogene oder gefällte Kieselsäuren, amorphe Silikate oder Metalloxide und röntgenopake Materialien, wie röntgenopake Gläser, Bariumsulfat oder Ytterbiumfluorid, und als Katalysatoren Dibenzoylperoxid, Dilaurylperoxid, Butylperoctat, Azobisisobutyronitril, Benzpinakol (für die Heißpolymerisation), Benzyl- bzw. Laurylperoxid zusammen mit Aminen (für die Kaltpolymerisation) bevorzugt werden. Außerdem kommen u.a. Benzophenon, Benzochinon sowie deren Derivate, $\alpha$-Diketone, Campherchinon (für die radikalische Polymerisation), Triphenyliodonium-, Triphenylsulfoniumsalze, Ferrocen-Derivate (für die kationische Photopolymerisation) und Broenstedt-, Lewis-Säuren bzw. Kombinationen von beiden (für die kationische Polymerisation) in Frage. Die zusätzlichen Monomere werden vorzugsweise aus der Gruppe bestehend aus Mono- oder Di(meth)acrylaten (wie z.B. Methylmethacrylat, Triethylenglycoldimethacrylat, Hexandioldimethacrylat, Dodecandioldimethacrylat, Bisphenol-A-dimethacrylat, Bisphenol-A-glycidylmethacrylat, Trimethyloylpropantrimethacrylat, Urethandimethacrylate), Epoxiden oder Vinyletherverbindungen ausgewählt.

Mit den erfindungsgemäßen bicycloaliphatischen 2-Methylen-1,3-dioxepanen werden somit Ausgangsstoffe zur Herstellung von Polymeren bzw. Copolymeren, vorzugsweise von Dentalmaterialien, zur Verfügung gestellt, die auf einfache Weise hergestellt werden können, nahezu geruchlos, destillierbar und leicht modifizierbar sind und sich sowohl bei Raumtemperatur radikalisch bzw. kationisch photopolymerisieren, thermisch mit und ohne Radikalinitiator, wie auch kationisch härten lassen.

Die vorliegende Erfindung wird nachfolgend anhand von Beispielen beschrieben.

**<u>Beispiele für die Herstellung und Verwendung bicycloaliphatischer 2-Methylen-1,3-dioxepane</u>**

**Beispiel 1:**

<u>4-Methylen-9,9-dimethyl-3,5,8,10-tetraoxa-bicyclo[5.3.0]decan</u> (<u>1</u>)

1. Stufe: <u>2-Brommethyl-4,7-dihydro-1,3-dioxepin</u>:

Eine Mischung aus 3,22 mol (635 g) 2-Bromacetaldehyddiethylacetal, 3, 16 mol (278 g) cis-2-Buten-1,4-diol und 6 g p-Toluolsulfonsäure (Katalysator) wurde in einem 2 l Destillationskolben vorgelegt und 10 Minuten bei Raumtemperatur gerührt. Anschließend destillierte man im Wasserstrahlvakuum bei einer Badtemperatur von ca. 200 °C das Produkt und Ethanol über. Das farblose, klare Destillat wurde anschließend im Wasserstrahlvakuum fraktioniert destilliert, wobei 2-Brommethyl-4,7-dihydro-1,3-dioxepin bei 108 bis 110 °C (22,61 mbar (17 mm Hg)) überging und 523,3 g farbloses, klares Produkt erhalten wurden (86 % Ausbeute).

| | | | | |
|---|---|---|---|---|
| $C_6H_9O_2Br$ (193,04 g/mol): | Gef.: | C 37,09 | H 4,77 | Br 41,59 |
| | Ber.: | C 37,33 | H 4,70 | Br 41,39 |

$^1$H-NMR ($CDCl_3$, ppm): 3,42 (d,2H,Br-$CH_2$-), 4,00-4,60 (m,4H;-$CH_2$-0-), 4,98 (t,1H,>CH-) und 5,62-577 (m,2H,=CH-).
$^{13}$C-NMR ($CDCl_3$, ppm): 31,4 (C2), 65,8 (C3), 102,2 (C1) und 129,2 (C4).

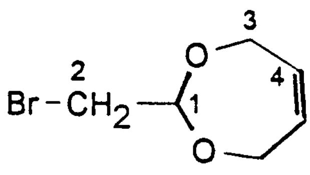

**2-Brommethyl-4,7-dihydro-1,3-dioxepin**

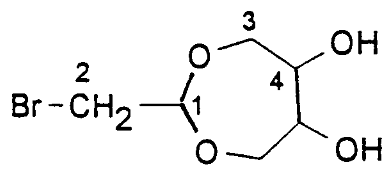

**2-Brommethyl-5,6-dihydroxy-1,3-dioxepan**

2. Stufe: <u>2-Brommethyl-5,6-dihydroxy-1,3-dioxenan</u>:

907 mmol (175 g) 2-Brommethyl-4,7-dihydro-1,3-dioxepin gelöst in 4,9 l Ethanol wurden auf -60 °C abgekühlt. Dazu tropfte man eine Lösung von 862 mmol Kaliumpermanganat (136,2 g) in 1,6 l Wasser über einen Zeitraum von 1,5 h so zu, daß die Innentemperatur -40 °C nicht überstieg. Man ließ über Nacht bei Raumtemperatur stehen, filtriert den Braunstein ab und engte die klare Lösung im Rotationsverdampfer (Wasserstrahlvakuum) soweit ein, bis eine Trübung einsetzte. Aus der zurückbleibenden Lösung fiel im Kühlschrank eine weiße kristalline Substanz aus, die aus Methylenchlorid umkristallisiert wurde. Nach dem Trocknen im Wasserstrahlvakuum wurden 194 g 2-Brommethyl-5,6-dihydroxy-1,3-dioxepan (32% Ausbeute) erhalten, wobei der Schmelzbereich bei 85 bis 95°C lag (bestimmt durch DSC).

| | | | | |
|---|---|---|---|---|
| $C_6H_{11}O_4Br$ (227,05 g/mol): | Gef.: | C 31,63 | H 4,81 | Br 35,34 |
| | Ber.: | C 31,74 | H 4,88 | Br 35,19 |

$^1$H-NMR ($CDCl_3$, ppm): 2,95 (s,2H,-OH), 3,30 (d,2H,-$CH_2$Br), 3,50-4,07 (m,6H, -$CH_2$-0-,>C<u>H</u>-OH) und 4,95 (t,1H,-0-C($CH_2$Br)<u>H</u>-0-).
$^{13}$C-NMR ($CDCl_3$, ppm): 33,4 (C2), 66,3 (C3), 71,2 (C4) und 100,1 (C1).

3. Stufe: <u>4-Brommethyl-9,9-dimethyl-3,5,8,10-tetraoxa-bicyclo [5.3.0]decan</u>:

Eine Mischung aus 0,93 mol (211 g) 2-Brommethyl-5,6-dihydroxy-1,3-dioxepan, 5,75 mol (422 ml) Aceton, 1,8 g p-Toluolsulfonsäure und 750 ml Methylenchlorid wurde unter Rückfluß erhitzt und das gebildete Wasser mittels eines Wasserabscheiders abgetrennt. Nach ca. 9 h zog man das Methylenchlorid im Rotationsverdampfer (Wasserstrahlvakuum) ab, und der Rückstand wurde im Feinvakuum fraktioniert destilliert. Man erhielt bei 89-92 °C (0,5 mbar) 231 g an 4-Brommethyl-9,9-dimethyl-3,5,8,10-tetraoxa-bicyclo[5.3.0]decan (93 % Ausbeute) als farblose Flüssigkeit, die bei Raumtemperatur teilweise kristalline Ausscheidungen aufwies und eine Isomerenmischung darstellte.

| | | | | |
|---|---|---|---|---|
| $C_9H_{15}O_4Br$ (267,12 g/mol): | Gef.: | C 40,50 | H 5,58 | Br 29,70 |
| | Ber.: | C 40,47 | H 5,66 | Br 29,91 |

$^{1}$H-NMR ($CDCl_3$, ppm): 1,25-1,65 (m,6H,-$CH_3$), 3,33-3,40 (m,2H,-$CH_2$Br), 3,66-4,45 (m,6H, -$CH_2$-0-,>CH-0-) und 5.08-5,11 (m, 1H,-0-C($CH_2$Br)H-0-).
$^{13}$C-NMR ($CDlC_3$, ppm): 24,9, 25,0, 27,2 und 27,6 (C6), 31,1 und 31,3 (C2), 66,6, 67,0 (C3), 75,8 und 76,6 (C4), 104,3 und 105,0 (C1), 108,3 und 110,1 (C5).

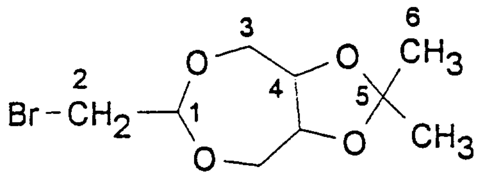

4-Brommethyl-9,9-dimethyl-3,5,8,10-tetraoxa-bicyclo[5.3.0]decan

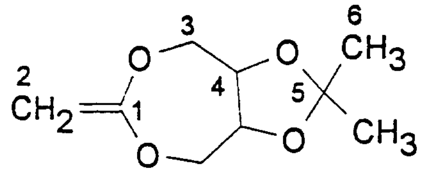

1

4. Stufe: 4-Methylen-9,9-dimethyl-3,5,8,10-tetraoxa-bicyclo[5.3.0]decan (1):

0,943 mol (105,8 g) Kalium-tert.-butylat wurden in 800 ml wasserfreiem THF gelöst. Dazu tropfte man eine Lösung von 865 mmol (231 g) 4-Brommethyl-9,9-dimethyl-3,5,8,10-tetraoxa-bicyclo[5.3.0]decan in 800 ml THF innerhalb von 90 Minuten unter Rühren zu und erwärmte anschließend 6 h unter Rückfluß. Das entstandene KBr wurde abfiltriert, und anschließend destillierte man das THF im Wasserstrahlvakuum ab. Der dunkelbraune Rückstand wird im Vakuum fraktioniert destilliert. Zwischen 45 bis 49 °C (0,2 mbar) gehen 111,2 g an farblos klarem 4-Methylen-9,9-dimethyl-3,5,8,10-tetraoxa-bicyclo[5.3.0]decan (Ausbeute 70 %) über.

| | | | |
|---|---|---|---|
| $C_9H_{14}0_4$ (186,21 g/mol): | Gef.: | C 57,84 | H 7,55 |
| | Ber.: | C 58,05 | H 7,58 |

$^{1}$H-NMR ($CDCl_3$, ppm): 1,40 und 1,53 (s,6H,-$CH_3$), 3,60 (s,2H,=$CH_2$), 3,72-4,30 (m,6H, -$CH_2$-0-,>CH-0-).
$^{13}$C-NMR ($CDCl_3$, ppm): 25,7 und 28,2 (C6), 66,5 (C3), 69,6 (C2), 74,8 (C4), 109,1 (C5) und 163, 1 (C1).

**Beispiel 2:**

4-Methylen-9-ethyl-9-methyl-3,5,8,10-tetraoxa-bicyclo[5.3.0]decan (2)

1. Stufe: 4-Brommethyl-9-ethyl-9-methyl-3,5,8,10-tetraoxa-bicyclo[5.3.0]decan

4-Brommethyl-9-ethyl-9-methyl-3,5,8,10-tetraoxa-bicyclo[5.3.0]decan wurde analog 4-Brommethyl-9,9-dimethyl-3,5,8,10tetraoxa-bicyclo[5.3.0]decan ausgehend von 198,2 mmol (45 g) 2-Brommethyl-5,6-dihydroxy-1,3-dioxepan und von 1,225 mol (88,3 g) Ethylmethylketon hergestellt, wobei die fraktionierte Destillation 46 g (82 % Ausbeute) im Bereich von 95 bis 100 °C (0,25 mbar) ergab.

| | | | | |
|---|---|---|---|---|
| $C_{10}H_{17}0_4$Br (281,15 g/mol): | Gef.: | C 42,66 | H 5,91 | Br 28,24 |
| | Ber.: | C 42,72 | H 6,09 | Br 28,42 |

$^{13}$H-NMR ($CDCl_3$, ppm): 0,93 (t,3H,$CH_3$-$CH_2$-), 1,30 und 1,38 (t,3H,-$CH_3$), 1,65 (q,2H,$H_3$C-$CH_2$-), 3,30 und 3,37 (s,2H,-$CH_2$Br), 3,63-4,60 (m,6H,-$CH_2$-0-,>CH-0-) und 4,75 und 4,77 (t,1 H,-0-C($CH_2$Br)H-0-).
$^{13}$C-NMR ($CDCl_3$, ppm): 7,9 und 8,7 (C8), 22,9 und 24,3 (C6), 31,4 und 32,8 (C2 und C7), 67,5 und 67,8 (C3), 75,5 und 76,2 (C4), 104,4 und 105, 1 (C1), 110,7 und 111,8 (C5).

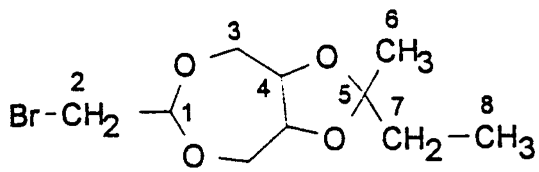 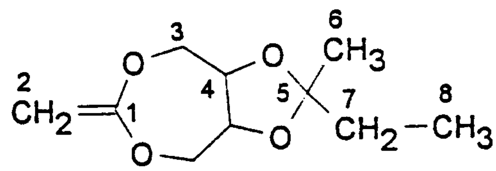

**4-Brommethyl-9-ethyl-9-methyl-3,5,8,10-tetraoxa-bicyclo[5.3.0]decan** 2

2. Stufe: 4-Methylen-9-ethyl-9-methyl-3,5,8,10-tetraoxa-bicyclo[5.3.0]decan (2):

4-Methylen-9-ethyl-9-methyl-3,5,8,10-tetraoxa-bicyclo[5.3.0]decan wurde analog 4-Methylen-9,9-dimethyl-3,5,8,10-tetraoxa-bicyclo[5.3.0]decan ausgehend von 142,3 mmol (40 g) 4-Brommethyl-9-ethyl-9-methyl-3,5,8,10-tetraoxa-bicyclo[5.3.0]decan hergestellt, wobei die fraktionierte Destillation 23 g (89 % Ausbeute) im Bereich von 53 bis 56 °C (0,2 mbar) ergab.

| | | | |
|---|---|---|---|
| $C_9H_{16}0_4$ (200,23 g/mol): | Gef.: | C 59,85 | H 7,93 |
| | Ber.: | C 59,99 | H 8,06 |

$^1$H-NMR ($CDCl_3$, ppm): 0,93 und 0,97 (t,3H,$C\underline{H}_3$-CH2-), 1,32 und 1,43 (t,3H, -CH), 1,65 (q,2H,$H_3$C-$C\underline{H}_2$-), 3,57 (s,2H,=$CH_2$), 3,80-4,33 (m,6H,-$CH_2$-0-,>CH-0-).
$^{13}$C-NMR ($CDCl_3$, ppm): 8,5 und 8,9 (C8), 22,9 und 25,3 (C6), 31,6 und 34,0 (C7), 68,4 und 69,6 (C2 und C3), 74,2 und 74,7 (C4), 110,9 und 111 ,34 (C5), 163,3 und 163,7 (C1).

**Beispiel 3:**

4-Methylen-9-methyl-9-vinyl-3,5,8,10-tetraoxa-bicyclo[5.3.0]decan (3)

1. Stufe: 4-Brommethyl-9-methyl-9-vinyl-3,5,8,10-tetraoxa-bicyclo[5.3.0]decan:

4-Brommethyl-9-methyl-9-vinyl-3,5,8,10-tetraoxa-bicyclo[5.3.0]decan wurde analog 4-Brommethyl-9,9-dimethyl-3,5,8, 10-tetraoxa-bicyclo[5.3.0]decan ausgehend von 220 mmol (50 g) 2-Brommethyl-5,6-dihydroxy-1,3-dioxepan und von 1,36 mol (95,5 g) Methylvinylketon hergestellt, wobei die fraktionierte Destillation 33,2 g 4-Brommethyl-9-methyl-9-vinyl-3,5,8,10-tetraoxa-bicyclo[5.3.0]decan (54 % Ausbeute) mit einem Siedepunkt im Bereich von 94 bis 101 °C (0,2 mbar) ergab.

$^1$H-NMR ($CDCl_3$, ppm): 1,42 und 1,47 (s,3H,$C\underline{H}_3$-), 3,32 und 3,35 (s,2H,-$CH_2$Br), 3,53-4,57 (m,6H,-$CH_2$-0-,>CH-0-), 4,80 (t,1H,-0-C($CH_2$Br)$\underline{H}$-0-), 5,07-5,50 (m,2H,=$CH_2$) und 5,80 (dd,1H,-CH=).

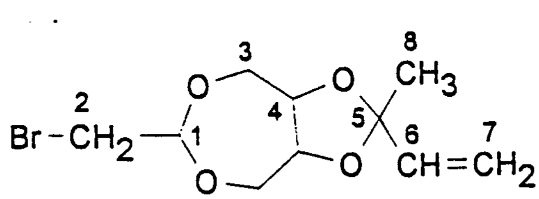 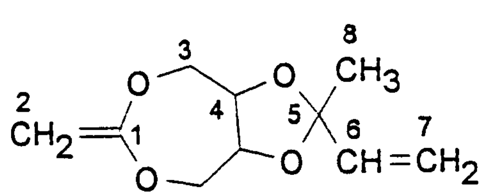

**4-Brommethyl-9-methyl-9-vinyl-3,5,8, 10-tetraoxa-bicyclo[5.3.0]decan** 3

2. Stufe: 4-Methylen-9-methyl-9-vinyl-3,5,8,10-tetraoxa-bicyclo[5.3.0]decan (3):

4-Methylen-9-methyl-9-vinyl-3,5,8,10-tetraoxa-bicyclo[5.3.0]decan wurde analog 4-Methylen-9,9-dimethyl-3,5,8,10-tetraoxa-bicyclo[5.3.0]decan ausgehend von 115,7 mmol (35 g) 4-Brommethyl-9-methyl-9-vinyl-3,5,8,10-tetraoxa-bicyclo[5.3.0)decan hergestellt, wobei die fraktionierte Destillation 10 g (43,2 % Ausbeute) bei 62 °C (0,1 mbar) ergab.

$^{1}$H-NMR ($CDCl_3$, ppm): 1,42 und 1,52 (s,3H,C$\underline{H}_3$-), 3,22 und 3,58 (s,2H,=$CH_2$), 3,80-4,37 (m,6H,-$CH_2$-0-,>CH-0-), 5,03-5,58 (m,2H,=$CH_2$) und 5,80 (dd,1H,-CH=).
$^{13}$C-NMR ($CDCl_3$, ppm): 24,7, 24,9 und 26,5 (C8), 66,1, 66,5 und 69,6 (C3), 74,6 und 74,8 (C4), 107,6 und 108,7 (C5), 114,7 und 115,3 (C7), 137,8, 138,4 und 139,8 (C6) und 163,1 und 163,6 (C1).

**Beispiel 4:**

4-Methylen-9-ethyl-3,5,8,10-tetraoxa-bicyclo[5.3.0]decan (4)

1. Stufe: 4-Brommethyl-9-ethyl-3,5,8,10-tetraoxa-bicyclo[5.3.0]decan

4-Brommethyl-9-ethyl-3,5,8,10-tetraoxa-bicyclo[5.3.0]decan wurde analog 4-Brommethyl-9,9-dimethyl-3,5,8,10-tetraoxa-bicyclo[5.3.0]decan ausgehend von 132 mmol (30 g) 2-Brommethyl-5,6dihydroxy-1,3-dioxepan und von 0,816 mol (60 ml) Propionaldehyd hergestellt, wobei die fraktionierte Destillation 25 g (71 % Ausbeute) im Bereich von 100 bis 102 °C (0,4 mbar) ergab.

| | | | | |
|---|---|---|---|---|
| $C_9H_{15}0_4Br$ (267,12 g/mol): | Gef.: | C 42,48 | H 5,89 | Br 26,95 |
| | Ber.: | C 40,47 | H 5,66 | Br 29,91 |

$^{1}$H-NMR ($CDCl_3$, ppm): 0,83-1,13 (m,3H,C$\underline{H}_3$-CH2-), 1,30 und 1,52-1,87 (m,2H,$H_3$C-C$\underline{H}_2$-), 3,32 (s,2H,-$CH_2$Br), 3,57-4,43 (m,6H,-$CH_2$-0-,>CH-0-) und 4,70-5,00 (m,2H,-0-C($CH_2$Br)$\underline{H}$-0-, >C(C2H5)$\underline{H}$).
$^{13}$C-NMR ($CDCl_3$, ppm): 8,0 (C7), 26,9 (C6), 31,3 (C2), 67,1 (C3), 76,7 (C4), 105,4 und 106,0 (C1 und C5).

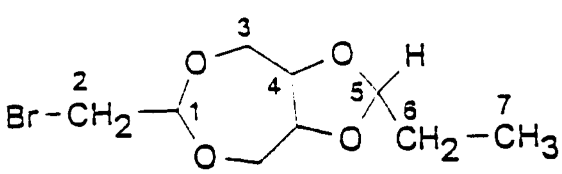

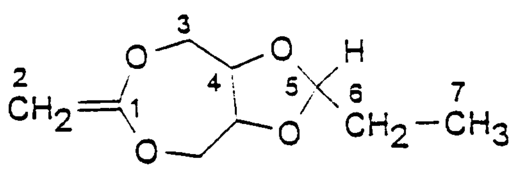

4-Brommethyl-9-ethyl-3,5,8,10-tetraoxa-bicyclo[5.3.0]decan 4

2. Stufe: 4-Methylen-9-ethyl-3,5,8,10-tetraoxa-bicyclo[5.3.0]decan (4):

4-Methylen-9-ethyl-3,5,8,10-tetraoxabicyclo[5.3.0]decan wurde analog 4-Methylen-9,9-dimethyl-3,5,8,10-tetraoxa-bicyclo[5.3.0]decan ausgehend von 131 mmol (35 g) 4-Brommethyl-9-ethyl-3,5,8,10-tetraoxa-bicyclo[5.3.0]decan hergestellt, wobei die fraktionierte Destillation im Bereich von 65 bis 68 °C (0,3 mbar) 12,5 g (51 % Ausbeute) ergab.

| | | | |
|---|---|---|---|
| $C_9H_{14}0_4$ (186,06 g/mol): | Gef.: | C 57,38 | H 7,48 |
| | Ber.: | C 58,05 | H 7,58 |

$^1$H-NMR ($CDCl_3$, ppm): 1,00 (t,3H,$C\underline{H}_3$-$CH_2$-), 1,53-1,90 (m,2H,$H_3C$-$C\underline{H}_2$-), 3,60 (s,2H,=$CH_2$), 3,80-4,30 (m,6H,-$CH_2$-0-,>CH-0-) und 4,93 (t,1H, >C($C_2H_5$)$\underline{H}$).

**Beispiel 5:**

Spiro[cyclohexan-1,1'-(6-methylen-2,5,7,10-tetraoxabicyclo[5,3,0]decan)] (22)

1. Stufe: Spiro[cyclohexan-1,1'-(6-brommethyl-2,5,7,10-tetraoxabicyclo[5.3.0]decan]:

Das Dioxan wurde analog 4-Brommethyl-9,9-dimethyl-3,5,8,10-tetraoxabicyclo[5.3.0]decan ausgehend von 220 mmol (50 g) 2-Brommethyl-5,6-dihydroxy-1,3-dioxepan und von 134 g (1,36 mol) Cyclohexanon hergestellt, wobei die fraktionierte Destillation 32,1 g Produkt (47 %) Ausbeute) im Bereich von 140 bis 155 °C (0,01 mbar) ergab.

| | | | | |
|---|---|---|---|---|
| $C_{12}H_{19}BrO_4$ (307,14 g/mol) | Gef.: | C 48,69 | H 6,46 | Br 24,87 |
| | Ber.: | C 46,93 | H 6,23 | Br 26,05 |

$^1$H-NMR($CDCl_3$); ppm; Varian 90 MHz):
1,6 (m, $CH_2$ Cyclohexyl), 3,3 (dd, $CH_2Br$), 3,6 - 4,5 (viele m, CHO und $CH_2O$), 4,8 (t, O-CH-O).
$^{13}$C-NMR ($CDCl_3$, ppm; Bruker 300 MHz):
110,7 (C2), 105,0 (C5), 75,6 (C4), 67,2 (C3), 37,0 (C1), 34,4 (C6), 31,1 (C8), 25,2 (C7)

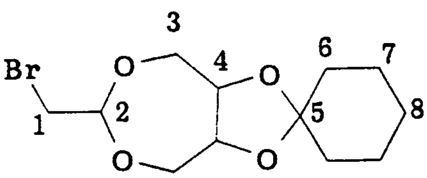

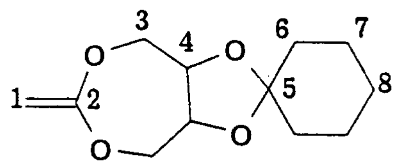

Spiro[cyclohexan-1,1'-(6-brommethyl-2,5,7,10-tetraoxabicyclo[5.3.0]decan]    22

2. Stufe: Spiro[cyclohexan-1,1'-(6-methylen-2,5,7,10-tetraoxabicyclo[5,3,0]decan)] (22):

Das Dioxepan wurde analog 4-Methylen-9,9-dimethyl-3,5,8,10-tetraoxabicyclo[5.3.0]decan ausgehend von 115,7 mmol (35 g) Spiro[cyclohexan-1,1'-(6-brommethyl-2,5,7,10-tetraoxabicyclo[5,3,0]decan)] hergestellt, wobei die fraktionierte Destillation 8,5 g (38,5 % Ausbeute) bei 98 bis 104 °C (0,1 mbar) ergab.

| | | | |
|---|---|---|---|
| $C_{12}H_{18}O_4$ (226,12 g/mol) | Gef.: | C 63,59 | H 8,01 |
| | Ber.: | C 64,74 | H 8,02 |

$^1$H-NMR ($CDCl_3$, ppm; Varian 90 MHz): 1,7 (m, $CH_2$ Cyclohexyl) 3,6 (s, =$CH_2$), 3,8 - 4,4 m, CHO und $CH_2O$)
$^{13}$C-NMR ($CDCl_3$; ppm; Bruker 300 MHz): 163,5 (C2), 109,7 (C5), 74,4 (C4), 69,5 (C1), 66,7 (C3), 38,1, 35,1, 27,0, 25,0, 24,1 (cyclohexyl)

**Beispiel 6:**

4-Methylen-9-phenyl-3,5,8,10-tetraoxabicyclo[5.3.0]decan (8, R=H)

1. Stufe: 4-Brommethyl-9-phenyl-3,5,8,10-tetraoxabicyclo[5.3.0]decan:

4-Brommethyl-9-phenyl-3,5,8,10-tetraoxabicyclo[5.3.0]decan wurde analog 4-Brommethyl-9,9-dimethyl-3,5,8,10-tetraoxabicyclo[5.3.0]decan aus 64,7 g 2-Brommethyl-5,6-dihydroxy-1,3-dioxepan (285 mmol) und 187,0 g Benzaldehyd (1760 mmol) in Chloroform hergestellt. Die Reaktionszeit betrug 8 Tage, wobei an jedem Tag eine erneute Spatelspitze Toluolsulfonsäure zugegeben wurde.

Destillation im Feinvakuum ($Sdp._{p0.1\ mbar}$ : 130 bis 169 °C) ergab 69,7 g (= 77,7 % Ausbeute) einer neongelben Flüssigkeit.

$^1H$-NMR ($CDCl_3$); ppm; Varian 90 MHz):
7,5 (-, aromat), 3,3 (dd, $CH_2Br$), 3,6 - 4,5 (viele m, CHO und $CH_2O$), 4,8 (t, O-CH-O).
$^{13}C$-NMR ($CDCl_3$, ppm; Bruker 300 MHz):
31,7 (C1), 103,8 (C2), 67,2 (C3), 73,9 (C4), 104,5 (C5), 125,4, 128,4, 13,6 und 143,4 (arom. C)
IR (KBr, $cm^{-1}$): 3036, 2882, 1700, 1599

| | | | | | |
|---|---|---|---|---|---|
| $C_{13}H_{15}BrO_4$ (315 g/mol) | Ber.: | C 49,52 | H 4,76 | Br 25,40 | O 20,32 |
| | Gef.: | C 54,69 | H 4,91 | Br 18,05 | O 22,35 |

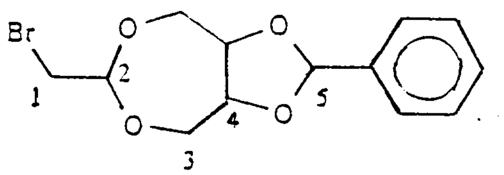

4-Brommethyl-9-phenyl-3,5,8,10-tetraoxabicyclo[5.3.0]decan

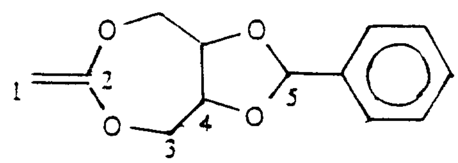

8

2. Stufe: 4-Methylen-9-phenyl-3,5,8,10-tetraoxabicyclo[5.3.0]decan (8):

69,6 g 4-Brommethyl-9-phenyl-3,5,8,10-tetraoxabicyclo[5.3.0]decan wurden mit 31,1 g Kalium-tert.-butoxid, wie in Beispiel 1 (4-Methylen-9,9-dimethyl-3,5,8,10-tetraoxabicyclo[5.3.0]decan) beschrieben, umgesetzt. Nach üblichen Reinigungsschritten wurde das Produkt im Feinvakuum ($Sdp._{p0,5\ mbar}$ : 98 bis 132 °C) destilliert. Beim Abkühlen bildete sich ein weißer Feststoff (Smp : 64 °C). Ausbeute 27,1 g = 44,7 %.

$^1H$-NMR ($CDCl_3$), ppm; Varian 90 MHz): 3,6 (s, $=CH_2$), 3,8 - 4,4 (m, CHO und $CH_2O$)
$^{13}C$-NMR ($CDCl_3$); ppm; Bruker 300 MHz):
69,9 (C1), 163,1 (C2), 66,0 (C3), 75,4 (C4), 103,3 (C5), 126,6, 128,3, 128,9 und 129,6 (arom. C)

| | | | | |
|---|---|---|---|---|
| $C_{13}H_{14}O_4$ (234 g/mol) | Ber.: | C 66,70 | H 5,97 | O 27,33 |
| | Gef.: | C 66,79 | H 6,04 | O 27,17 |

Homopolymerisation von 4-Methylen-9,9-dimethyl-3,5,8,10-tetraoxabicyclo[5.3.0]decan (1)

**Beispiel 7:**

Polymerisation in Substanz

1,0 g Monomer, vermischt mit 2,5 mol % AIBN, wurden 16 Stunden bei 120 °C belassen. Das weiße Produkt löste man in THF und fällte in Hexan um. Es resultierten 54,5 % fällbares Produkt mit einem $M_n$(GPC) von 7100 g/mol.

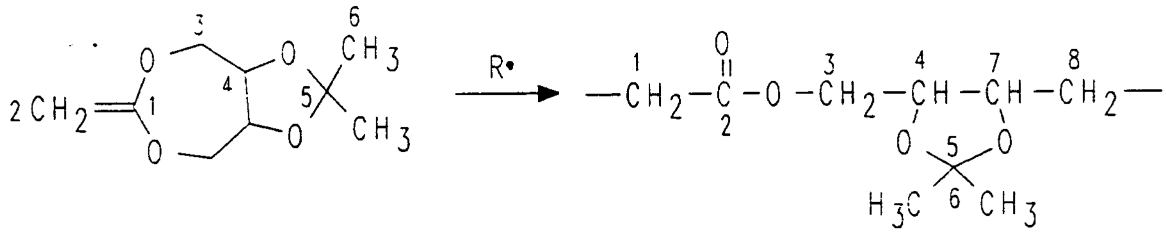


Poly(MA-29) - umgefällt

$^1$H-NMR (90 MHz. ppm): 1,19-1,30 (m): $CH_3$-Endgruppen; 1,35 (s,3H) und 1,42 (s,3H): -$CH_3$; 1,70-1,98 (m,2H): >CH-C$\underline{H_2}$-; 2,37-2,77 (m2H): -$CH_2$-CO-; 3,93-4,45 (m,4H): >CH- und -CO-O-$CH_2$-.
$^{13}$C-NMR ($CDCl_3$), ppm): 14,7 ($CH_3$-Endgruppen), 24,6 und 28,0 (C6), 30,8 (C8), 63,0 und 63,3 (C3), 72,5, 75,0, 75,2 und 75,7 (C4 und C7), 108,3 und 108,7 (C5) und 172,7 (C2).
IR (KBr, $cm^{-1}$): 1740 (C=O).

**Beispiel 8:**

Kationische ringöffnende Polymerisation

0,80 Gew.-% Cyacure UVI 6974 (Triarylsulfoniumhexafluoroantimonat, 50 %-ig in Propylencarbonat) wurden in 4,-Methylen-9,9-dimethyl-3,5,8,10-tetraoxa-bicyclo[5.3.0]decan gelöst. Ein 500 μm dicker Film dieses Gemisches wurde unter Luftausschluß auf beiden Seiten je 3 Minuten mit einer UV/VIS-Lampe (Spectramat® der Firma Ivoclar, Wellenlängenbereich 260 bis über 600 nm) bestrahlt. Das erhaltene Polymer ist z.B. in Hexan schwer löslich. Der lösliche Anteil weist die in Beispiel 7 angegebenen $^1$H-NMR und $^{13}$C-NMR-Spektren auf.

**Beispiel 9:**

7,80 Gew.-% Dioxepan aus Beispiel 1 wurden mit 31,60 % propoxyliertem Bisphenol-A-dimethacrylat, 41,28 % pyrogenem Kieselgel, 18,70 g $YbF_3$, 0,24 % Campherchinon, 0,23 % N,N-Diethyl-3,5-ditert.-butylanilin, 0,14 % Sodapaste und 0,01 % Butylhydroxytoluol zu einer homogenen Masse verknetet. Diese Masse wurde in einem Formkörper unter Luftausschluß 3 Minuten mit einer UV/VIS-Lampe (Spectramat® der Firma Ivoclar, Wellenlängenbereich 260 bis über 600 nm) bestrahlt. Der gemessene Polymerisationsschrumpf betrug 1,9 %. Zum Vergleich wurde bei einem Komposit der gleichen Zusammensetzung, das 1,10-Decandioldimethacrylat anstelle der Dioxepankomponente enthält, ein Volumenschrumpf von 4,2 % bestimmt.

**Patentansprüche**

1. Bicycloaliphatische 2-Methylen-1,3-dioxepane der allgemeinen Formel

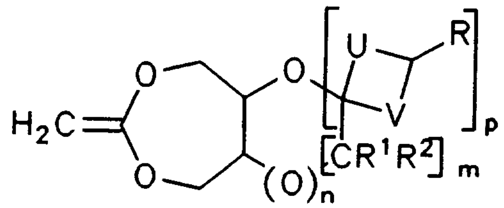

(I),

wobei

n gleich 1 ist, und
p gleich 0 und m gleich 1 ist, oder
p gleich 1 und m gleich 0 ist,
U und V jeweils Sauerstoff oder $(CH_2)_q$ bedeuten, wobei q eine ganze Zahl im Bereich von 0 bis 3 ist,
R, $R^1$ und $R^2$ jeweils für Wasserstoff, eine Alkylgruppe, eine Alkenylgruppe, eine Arylgruppe oder einen cycloaliphatischen Rest stehen und jeweils einen weiteren Substituenten aus der Gruppe bestehend aus Alkyl, Vinyl, Acryl, Acryloxy, Methacryl, Methacryloxy, $COOR^3$, $CONR^3_2$ und $OR^3$ enthalten können und wobei $R^3$ für eine Alkylgruppe und/oder Arylgruppe steht, wobei einer der Reste R, $R^1$ oder $R^2$ mit einem zweiten 2-Methylen-1,3-dioxepanring der Formel (I) verknüpft sein kann oder $R^1$ und $R^2$ für eine Methylengruppe steht.

**2.** Dioxepan nach Anspruch 1, dadurch gekennzeichnet, daß n gleich 1 und p gleich 0 ist und der allgemeinen Formel

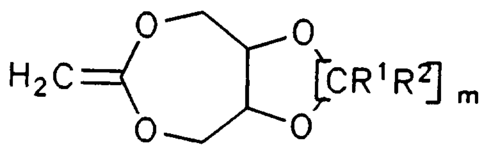

(II)

entspricht, wobei m, q, R, $R^1$, $R^2$ und $R^3$ die oben angegebene Bedeutung haben.

**3.** Dioxepan nach Anspruch 1, dadurch gekennzeichnet, daß n gleich 1 und p gleich 1 ist und der allgemeinen Formel

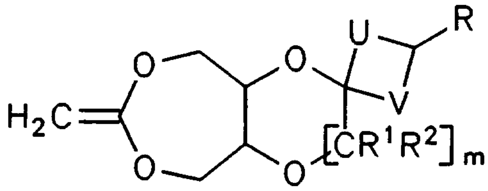

(III)

entspricht, wobei m, q, U, V, R, $R^1$, $R^2$ und $R^3$ die oben angegebene Bedeutung haben.

**4.** Dioxepan nach Anspruch 2, dadurch gekennzeichnet, daß m gleich 1 ist und $R^1$ und $R^2$ aus der Gruppe bestehend aus Wasserstoff, Methyl, Ethyl, Cyclohexyl, Norborn-1-en-5-yl und Phenyl, ausgewählt ist, wobei $R^1$ und $R^2$ jeweils gleich oder verschieden sein können und einen weiteren Substituenten R tragen können, der die oben angegebene Bedeutung hat, oder $R^1$ und $R^2$ für eine Methylengruppe stehen.

**5.** Dioxepan nach Anspruch 2, dadurch gekennzeichnet, daß m gleich 1 ist, $R^1$ Wasserstoff bedeutet und $R^2$ für einen Substituenten der Formel $(CH_2)_rR$ steht, wobei r eine ganze Zahl im Bereich von 0 bis 4 ist und R aus der Gruppe bestehend aus Vinyl, Acryloxy und Methacryloxy ausgewählt ist.

**6.** Dioxepan nach Anspruch 2, dadurch gekennzeichnet, daß m gleich 1 ist, $R^1$ Wasserstoff und $R^2$ Phenyl bedeutet, wobei die Phenylgruppe in meta- oder para-Position durch eine zweite 2-Methylen-1,3,-dioxepangruppe der allgemeinen Formel (II) substituiert ist, bei der n und m gleich 1 sind, $R^1$ Wasserstoff bedeutet und $R^2$ die Verknüpfungsstelle zur Phenylgruppe darstellt.

**7.** Dioxepan nach Anspruch 3, dadurch gekennzeichnet, daß m gleich 0 ist, U und V jeweils $CH_2CH_2$ bedeuten und R für Wasserstoff steht.

**8.** Dioxepan nach Anspruch 3, dadurch gekennzeichnet, daß m gleich 0 ist, U und V jeweils $CH_2CH_2$ bedeuten und R für eine zweite 2-Methylen-1,3,-dioxepangruppe der allgemeinen Formel (II) steht, die in Form einer Spiroverknüpfung an dem $R^1$ und $R^2$ tragenden Kohlenstoff mit dem U und V enthaltenden Ring verbunden ist, wobei $R^1$ und $R^2$ der zweiten 2-Methylen-1,3-dioxepangruppe wegfallen und n und m gleich 1 sind.

**9.** Verwendung des 2-Methylen-1,3-dioxepans nach einem der Ansprüche 1 bis 8 zur Herstellung von Polymeren und/oder Copolymeren.

**10.** Verwendung nach Anspruch 9, wobei die Herstellung der Polymere durch Bestrahlung, thermisch oder mit Hilfe von Radikalinitiatoren oder kationischen Initiatoren erfolgt.

**11.** Verwendung nach den Ansprüchen 9 oder 10 zur Herstellung von Kompositmaterialien.

**12.** Verwendung nach Anspruch 11, dadurch gekennzeichnet, daß zusätzlich Füllstoffe, zusätzliche Monomere als Vernetzer und/oder Katalysatoren verwendet werden.

**13.** Verwendung nach Anspruch 12, dadurch gekennzeichnet, daß die Füllstoffe aus der Gruppe bestehend aus Silikaten, Gläsern oder Metalloxiden ausgewählt sind.

**14.** Verwendung nach Anspruch 12 oder 13, dadurch gekennzeichnet, daß die zusätzlichen Monomere aus der Gruppe bestehend aus Acrylaten, Methacrylaten, Diacrylaten, Dimethacrylaten, Vinylethern und Epoxiden ausgewählt sind.

**15.** Verwendung nach einem der Ansprüche 12 bis 14, dadurch gekennzeichnet, daß die Katalysatoren aus der Gruppe bestehend aus radikalischen und kationischen Initiatoren oder Initiatorsystemen, radikalischen und kationischen Photoinitiatoren ausgewählt sind.

**16.** Verwendung nach den Ansprüchen 9 bis 15 zur Herstellung von Dentalmaterialien.

**17.** Verwendung nach Anspruch 16, wobei das Dentalmaterial ein temporäres Füllungsmaterial oder ein Dentaladhäsiv ist.

## Claims

**1.** Bicycloaliphatic 2-methylene-1,3-dioxepanes having the general formula

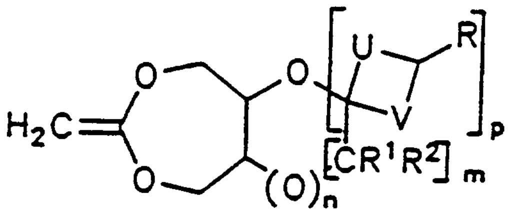

(I),

where

n is 1 and p is 0 and m is 1, or
p is 1 and m is 0,
U and V each mean oxygen or $(CH_2)_q$, where q is an integer from 0 to 3,
R, $R^1$ and $R^2$ each stand for hydrogen, an alkyl group, an alkenyl group, an aryl group or a cycloaliphatic radical and may each contain a further substituent from the group consisting of alkyl, vinyl, acryloyl, acryloyloxy, methacryloyl, methacryloyloxy, $COOR^3$, $CONR^3_2$ and $OR^3$, and where $R^3$ stands for an alkyl group and/or aryl group, where one of the radicals R, $R^1$ or $R^2$ may be linked with a second 2-methylene-1,3-dioxepane ring having the formula (I), or $R^1$ and $R^2$ stands for a methylene group.

2. Dioxepane according to Claim 1, characterized in that n is 1 and p is 0 and it corresponds to the general formula

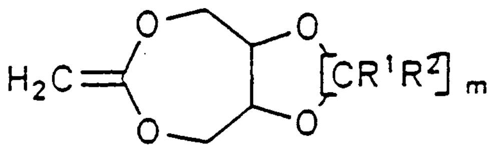

(II),

where m, q, R, $R^1$, $R^2$ and $R^3$ have the meaning given above.

3. Dioxepane according to Claim 1, characterized in that n is 1 and p is 1 and it corresponds to the general formula

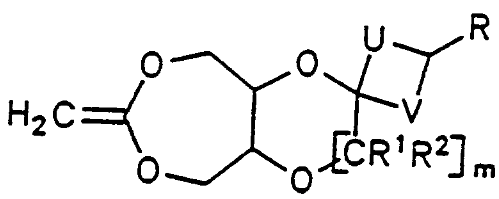

(III),

where m, q, U, V, R, $R^1$, $R^2$ and $R^3$ have the meaning given above.

4. Dioxepane according to Claim 2, characterized in that m is 1 and $R^1$ and $R^2$ is chosen from the group consisting of hydrogen, methyl, ethyl, cyclohexyl, norborn-1-en-5-yl and phenyl, where $R^1$ and $R^2$ may each be the same or different and may bear another substituent R which has the meaning given above, or $R^1$ and $R^2$ stand for a methylene group.

5. Dioxepane according to Claim 2, characterized in that m is 1, $R^1$ means hydrogen and $R^2$ stands for a substituent having the formula $(CH_2)_rR$, where r is an integer from 0 to 4 and R is chosen from the group consisting of vinyl, acryloyloxy and methacryloyloxy.

6. Dioxepane according to Claim 2, characterized in that m is 1, $R^1$ means hydrogen and $R^2$ means phenyl, where the phenyl group is substituted in the meta or para position by a second 2-methylene-1,3-dioxepane group having the general formula (II), in which n and m are equal to 1, $R^1$ means hydrogen and $R^2$ is the linkage point to the phenyl group.

7. Dioxepane according to Claim 3, characterized in that m is 0, U and V each mean $CH_2CH_2$ and R stands for hydrogen.

8. Dioxepane according to Claim 3, characterized in that m is 0, U and V each mean $CH_2CH_2$ and R stands for a second 2-methylene-1,3-dioxepane group having the general formula (II) which is connected, in the form of a spiro linkage at the carbon bearing $R^1$ and $R^2$, to the ring containing U and V, where $R^1$ and $R^2$ of the second 2-methylene-1,3-dioxepane group are absent and n and m are equal to 1.

9. Use of the 2-methylene-1,3-dioxepane according to one of Claims 1 to 8 for the preparation of polymers and/or copolymers.

10. Use according to Claim 9, where the preparation of the polymers takes place by irradiation, thermally, or with the aid of radical initiators or cationic initiators.

11. Use according to Claims 9 or 10 for the preparation of composite materials.

12. Use according to Claim 11, characterized in that, in addition, fillers, additional monomers as crosslinking agents, and/or catalysts are used.

13. Use according to Claim 12, characterized in that the fillers are chosen from the group consisting of silicates, glasses or metal oxides.

14. Use according to Claim 12 or 13, characterized in that the additional monomers are chosen from the group consisting of acrylates, methacrylates, diacrylates, dimethacrylates, vinyl ethers and epoxides.

15. Use according to one of Claims 12 to 14, characterized in that the catalysts are chosen from the group consisting of radical and cationic initiators or initiator systems, radical and cationic photoinitiators.

16. Use according to Claim 9 to 15 for the preparation of dental materials.

17. Use according to Claim 16, where the dental material is a temporary filling material or a dental adhesive.

**Revendications**

1. 2-Méthylène-1,3-dioxépanes bicycloaliphatiques de formule générale

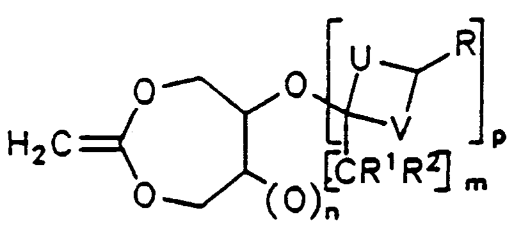

(I),

dans laquelle

n est égal à 1, et

p est égal à 0 et m à 1, ou

p est égal à 1 et m à 0,

U et V représentent chacun un atome d'oxygène ou un groupe $(CH_2)_q$, où q est un nombre entier situé dans l'intervalle de 0 à 3,

R, $R^1$ et $R^2$ sont chacun un atome d'hydrogène, un groupe alkyle, un groupe alcényle, un groupe aryle ou un radical cycloaliphatique, et peuvent chacun contenir un autre substituant appartenant au groupe constitué d'un alkyle, d'un vinyle, d'un acryle, d'un acryloxy, d'un méthacryle, d'un méthacryloxy, de $COOR^3$, de $CONR^3_2$ et de $OR^3$, et où $R^3$ est un groupe alkyle et/ou un groupe aryle, où l'un des radicaux R, $R^1$ ou $R^2$ peut être lié à un deuxième noyau 2-méthylène-1,3-dioxépane de formule (I), ou bien $R^1$ et $R^2$ sont un groupe méthylène.

**2.** Dioxépane selon la revendication 1, caractérisé en ce que n est égal à 1 et p à 0, et qui est conforme à la formule générale

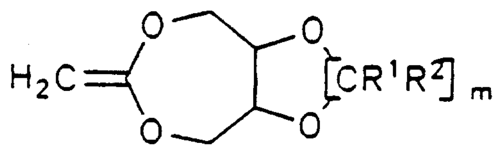

(II)

dans laquelle m, q, R, $R^1$, $R^2$ et $R^3$ ont la signification indiquée ci-dessus.

**3.** Dioxépane selon la revendication 1, caractérisé en ce que n est égal à 1 et p à 1, et qui est conforme à la formule générale

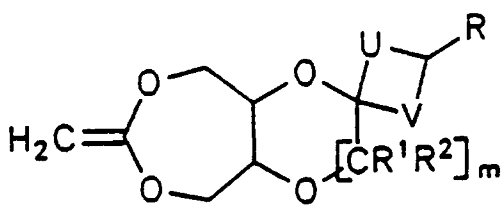

(III)

dans laquelle m, q, U, V, R, $R^1$, $R^2$ et $R^3$ ont la signification indiquée ci-dessus.

**4.** Dioxépane selon la revendication 2, caractérisé en ce que m est égal à 1, et en ce que $R^1$ et $R^2$ sont choisis dans le groupe constitué d'un atome d'hydrogène, d'un groupe méthyle, éthyle, cyclohexyle, norborn-1-èn-5-yle et phényle, où $R^1$ et $R^2$ peuvent être chacun identiques ou différents et peuvent porter un autre substituent R qui a la signification indiquée ci-dessus, ou bien $R^1$ et $R^2$ sont un groupe méthylène.

**5.** Dioxépane selon la revendication 2, caractérisé en ce que m est égal à 1, $R^1$ représente un atome d'hydrogène, et $R^2$ est un substituant de formule $(CH_2)_rR$, où r est un nombre entier situé dans l'intervalle de 0 à 4, et R est choisi dans le groupe constitué d'un vinyle, d'un acryloxy et d'un méthacryloxy.

**6.** Dioxépane selon la revendication 2, caractérisé en ce que m est égal à 1, $R^1$ représente un atome d'hydrogène et $R^2$ un groupe phényle, où le groupe phényle est substitué en position méta ou para par un deuxième groupe 2-méthylène-1,3-dioxépane de formule générale (II) dans laquelle n et m sont égaux à 1, $R^1$ représente un atome d'hydrogène et $R^2$ représente le cite de liaison au groupe phényle.

**7.** Dioxépane selon la revendication 3, caractérisé en ce que m est égal à 0, U et V représentent chacun $CH_2CH_2$, et R est un atome d'hydrogène.

8. Dioxépane selon la revendication 3, caractérisé en ce que m est égal à 0, U et V représentent chacun $CH_2CH_2$, et R est un deuxième groupe 2-méthylène-1,3-dioxépane de formule générale (II), qui est relié sous la forme d'une liaison spiro à l'atome de carbone portant $R^1$ et $R^2$, au noyau contenant U et V, où $R^1$ et $R^2$ du deuxième groupe 2-méthylène-1,3-dioxépane sont supprimés, et n et m sont égaux à 1.

9. Utilisation du 2-méthylène-1,3-dioxépane selon l'une des revendications 1 à 8, pour la préparation de polymères et/ou de copolymères.

10. Utilisation selon la revendication 9, où la préparation des polymères a lieu par irradiation, par voie thermique ou à l'aide d'initiateurs radicalaires ou d'initiateurs cationiques.

11. Utilisation selon les revendications 9 ou 10, pour la préparation de matériaux composites.

12. Utilisation selon la revendication 11, caractérisée en ce qu'on utilise en plus des matériaux de chargement, des monomères supplémentaires en tant d'agents de réticulation et/ou des catalyseurs.

13. Utilisation selon la revendication 12, caractérisée en ce que les matériaux de chargement sont choisis dans le groupe constitué des silicates, des verres ou des oxydes métalliques.

14. Utilisation selon la revendication 12 ou 13, caractérisée en ce que les monomères supplémentaires sont choisis dans le groupe constitué des acrylates, des méthacrylates, des diacrylates, des diméthylacrylates, des éthers vinyliques et des époxydes.

15. Utilisation selon l'une des revendications 12 à 14, caractérisée en ce que les catalyseurs sont choisis dans le groupe constitué des initiateurs ou systèmes d'amorçage radicalaires et cationiques, et des photo initiateurs radicalaires et cationiques.

16. Utilisation selon les revendications 9 à 15, pour préparer des matériaux dentaires.

17. Utilisation selon la revendication 16, où le matériau dentaire est un matériau de remplissage temporaire ou un adhésif dentaire.